# EUROPÄISCHE PATENTANMELDUNG

(11) **EP 0 664 117 A1**
(43) Veröffentlichungstag der Anmeldung: **26.07.1995**
(21) Anmeldenummer: 95100293.0
(22) Anmeldetag: 11.01.1995
(51) Int. Cl.: A61K 9/127, A61K 31/43

(54) **Liposomenlösungen**

(30) Priorität: 25.01.1994 CH 213/94
(71) Anmelder: F. HOFFMANN-LA ROCHE AG, CH-4002 Basel (CH)
(72) Erfinder: Fernex, Michel, F-68450 Durmenach (FR); Steffen, Hans, CH-4410 Liestal (CH); Supersaxo, Andreas, CH-4052 Basel (CH); Theil, Frank-Peter, D-79189 Bad Krozingen (DE)
(74) Vertreter: Grossner, Lutz, Dr.

(57) **Zusammenfassung**

Die Erfindung betrifft wässrige Liposomenlösungen, die einen β-Lactamasehemmer und, gegebenenfalls, ein β-Lactamantibiotikum enthalten.

## Beschreibung

Die vorliegende Erfindung betrifft wässrige Liposomenlösungen, die einen β-Lactamasehemmer enthalten.

Es wurde gefunden, dass die Verweildauer von β-Lactamasehemmern, wie Tazobactam, im Körper verlängert ist, wenn der β-Lactamasehemmer in Form einer wässrigen Liposomenlösung verabreicht wird. Es wurde weiterhin gefunden, dass die Verweildauer des in einer solchen Form verabreichten β-Lactamasehemmers im Plasma durch die Grösse der Liposomen beeinflusst werden kann.

Die Liposomen der erfindungsgemässen Kompositionen können aus Stoffen zusammengesetzt sein, die als Liposomenbildner an sich bekannt sind. Vorzugsweise bestehen die Liposomen aus Phosphatidylcholin wie Eilecithin oder Sojalecithin, und Phosphatidylglycerin und, gegebenenfalls, Cholesterin. Das Molverhältnis von Phosphatidylcholin: Phosphatidylglycerin is zweckmässig 10:0 - 1, vorzugsweise 10:1. Das Molverhältnis von Phosphatidylcholin:Cholesterin ist zweckmässig 10:0 - 5 vorzugsweise 10:5. Die Konzentration der Liposomenbildner (d.h. Phosphatidylcholin, Phosphatidylglycerin und Cholesterin) in der Liposomenlösung beträgt zweckmässig etwa 10-400 mg/ml, vorzugsweise etwa 100-250 mg/ml.

Der Ausdruck β-Lactamasehemmer bezeichnet Stoffe, die die enzymatische Aufspaltung des β-Lactamrings von β-Lactamantibiotika wie Penicillinen, Cephalosporinen, Monolactamen und Carbapenemen hemmen. Beispiele von β-Lactamasehemmern sind Tazobactam, Sulbactam und Clavulansäure. Weitere Beispiele von β-Lactamasehemmern sind die in der europäischen Patentpublikation A-0 508 234 beschriebenen Verbindungen, insbesondere die Verbindungen
Benzyl (1S,5R)-7-oxo-6-sulfo-2,6-diazabicyclo[3.2.0]heptan-2-carboxylat,
(1S,5R)-2-[(Z)-3-α-Acetamidocinnamoyl]-7-oxo-2,6-diazabicyclo[3.2.0]-heptan-6-sulfonsäure,
(R/S)-α-(1S,5R)-2-[2-carboxy-2-(3-thienyl)acetyl]-7-oxo-2,6-diazabicyclo-[3.2.0]heptan-6-sulfonsäure,
(1S,5R)-7-Oxo-2-(3-pyridylacetyl)-2,6-diazabicyclo[3.2.0]heptan-6-sulfonsäure,
(1S,5R)-2-[(R,S)-2-Indolylcarbonyl]-7-oxo-2,6-diazabicyclo[3.2.0]heptan-6-sulfonsäure,
(1S,5R)-2-[(R)-α-Hydroxyphenylacetyl]-7-oxo-2,6-diazabicyclo[3.2.0]heptan-6-sulfonsäure,
(1S,5R)-2-[(S)-α-Hydroxyphenylacetyl]-7-oxo-2,6-diazabicyclo[3.2.0]heptan-6-sulfonsäure,
R(α)-[[(1S,5R)-7-Oxo-6-sulfo-2,6-diazabicyclo[3.2.0]hept-2-yl]carbonyl]-benzylsulfat,
(1S,5R)-2-(2-Amino-4-thiazolglyoxyloyl)-7-oxo-2,6-diazabicyclo[3.2.0]heptan-6-sulfonsäure
(1S,5R)-7-Oxo-2-(D-2-phenylglycyl-6-sulfo-2,6-diazabicyclo[3.2.0]heptan-6-sulfonsäure,
(1S,5R)-7-Oxo-2-(L-2-phenylglycyl)-6-sulfo-2,6-diazabicyclo[3.2.0]heptan-6-sulfonsäure,
(1S,5R)-7-Oxo-2-L-tyrosyl-2,6-diazabicyclo[3.2.0]heptan-6-sulfonsäure,
(1S,5R)-2-[D-2-(p-Hydroxyphenyl)glycyl]-7-oxo-2,6-diazabicyclo[3.2.0]heptan-6-sulfonsäure,
(1S,5R)-7-Oxo-2-L-tryptophanyl-2,6-diazabicyclo]3.2.0]heptan-6-sulfonsäure,
(1S,5R)-2-](R oder S)-α-Amino-(2-thienyl)acetyl]-7-oxo-2,6-diazabicyclo[3.2.0]heptan-6-sulfonsäure
(1S,5R)-7-oxo-2-(Phenylcarbamoyl)-6-sulfo-2,6-diazabicyclo[3.2.0]heptan,
(1S,5R)-2-(Benzylcarbamoyl)-7-oxo-6-sulfo-2,6-diazabicyclo[3.2.0]heptan,
(1S,5R)-7-Oxo-2-(N-phenylglycyl)-2,6-diazabicyclo[3.2.0]heptan-6-sulfonsäure,
(1S,5R)-2-[(E)-3-(2-Furyl)acryloyl]-7-oxo-2,6-diazabicyclo[3.2.0]heptan-6-sulfonsäure,
(1S,5R)-2-[N-(m-Aminophenyl)glycyl]-7-oxo-2,6-diazabicyclo[3.2.0]heptan-6-sulfonsäure,
(1S,5R)-2-[[(1-Methyl-1H-tetrazol-5-yl)thio]acetyl]-7-oxo-2,6-diazabicyclo[3.2.0]heptan-6-sulfonsäure,
(1S,5R)-2-[[(1H-benzotriazol-1-yl)thio]acetyl]-7-oxo-2,6-diazabicyclo[3.2.0]heptan-6-sulfonsäure, und
(1S,5R)-2-[(E)-3-(3-Indolyl)acryloyl]-7-oxo-2,6-diazabicyclo]3.2.0]heptan-6-sulfonsäure,
sowie die pharmazeutisch verträglichen Salze dieser Verbindungen.

Weitere β-Lactamasehemmer, die in den erfindungsgemässen Liposomenlösungen enthalten sein können, sind die Verbindungen der allgemeinen Formel I, 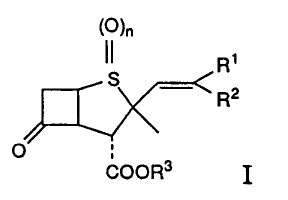
worin eines von R¹ und R² -COR⁴, -CN, -CH₂OR⁵ oder -SO₂R⁶ und das andere H, -COR⁴, -CN, -CH₂OR⁵ oder -SO₂R⁶ oder niederes Alkyl;
R³ = H, niederes Alkyl, Aryl-alkyl, Allyl oder einen in vivo abspaltbaren Rest;
R⁴ = H, niederes Alkyl, niederes Alkoxy, Benzyloxy, amino oder niederes Alkylamino;
R⁵ = H oder -CONH₂;
R⁶ = niederes Alkyl und
n = 0,1 oder 2 bedeuten
sowie die pharmazeutisch verträglichen Salze dieser Verbindungen, insbesondere die Verbindungen
(E/Z)-(2S,3S,5R)-3-(2-Cyanoethenyl)-3-methyl-7-oxo-4-thia-1-aza-bicyclo[3.2.0]heptan-2-carbonsäure-benzhydrylester,
(E)-(2S,3S,5R)-3-(2-Cyanoethenyl)-3-methyl-4,4,7-trioxo-4-thia-1-aza-bicyclo[3.2.0]heptan-2-carbonsäure-benzhydrylester,
(Z)-(2S,3S,5R)-3-(2-Cyanoethenyl)-3-methyl-4,4,7-trioxo-4-thia-1-aza-bicyclo[3.2.0]heptan-2-carbonsaure-benzhydrylester,
(E)-(2S,3S,5R)-3-(2-Cyanoethenyl)-3-methyl-4,4,7-trioxo-4-thia-1-aza-bicyclo[3.2.0]heptan-2-carbonsäure-Natriumsalz,
(Z)-(2S,3S,5R)-3-(2-Cyanoethenyl)-3-methyl-4,4,7-trioxo-4-thia-1-aza-bicyclo[3.2.0]heptan-2-carbonsäure-Natriumsalz.
(E/Z)-(2S,3S,5R)-3-(2-Carbamoyl-vinyl)-3-methyl-7-oxo-4-thia-1-aza-bicyclo[3.2.0]heptan-2-carbonsäure-benzhydrylester,
(E)-(2S,3S,5R)-3-(2-Carbamoyl-vinyl)-3-methyl-4,4,7-trioxo-4-thia-1-aza-bicyclo[3.2.0]heptan-2-carbonsäure-benzhydrylester,
(Z)-(2S,3S,5R)-3-(2-Carbamoyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Carbamoyl-vinyl)-3-methyl-4,4,7-trioxo-4-thia-1-aza-bicyclo[3.2.0]heptan-2-carbonsäure-Natriumsalz,
(Z)-(2S,3S,5R)-3-(2-Carbamoyl-vinyl)-3-methyl-4,4,7-trioxo-4-thia-1-aza-bicyclo[3.2.0]heptan-2-carbonsäure-Natriumsalz,
(E)-(2S,3S,5R)-3-(2-Ethoxycarbonyl-vinyl)-3-methyl-7-oxo-4-thia-1-aza-bicyclo[3.2.0]heptan-2-carbonsäure-benzhydrylester,
(E)-(2S,3S,5R)-3-(2-Ethoxycarbonyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Ethoxycarbonyl-vinyl)-3-methyl-4,4,7-trioxo-4-thia-1-aza-bicyclo[3.2.0]heptan-2-carbonsäure-Natriumsalz,
(E)-(2S,3S,5R)-3-(2-Benzyloxycarbonyl-vinyl)-3-methyl-7-oxo-4-thia-1-aza-bicyclo[3.2.0]heptan-2-carbonsäure-benzhydrylester,
(E)-(2S,3S,5R)-3-(2-Benzyloxycarbonyl-vinyl)-3-methyl-4,4,7-trioxo-4-thia-1-aza-bicyclo[3.2.0]heptan-2-carbonsäure-benzhydrylester,
(E)-(2S,3S,5R)-3-(2-Benzyloxycarbonyl-vinyl)-3-methyl-4,4,7-trioxo-4-thia-1-aza-bicyclo[3.2.0]heptan-2-carbonsäure-Natriumsalz.

Die Konzentration des β-Lactamasehemmers in den Liposomen hängt vom spezifischen β-Lactamasehemmer ab und muss individuell ermittelt werden. Zu hohe Wirkstoffkonzentrationen können nach Verabreichung der Liposomen in die Blutbahn oder ins Gewebe zum Platzen der Liposomen führen (osmotische Effekte). Beispielsweise beträgt die Konzentration von Tazobactam in den Liposomen zweckmässig nicht mehr als 0.2 mg / mg Liposomenbildner, vorzugsweise beträgt sie etwa 0.1 bis 0.15 mg/mg Liposomenbildner.

Es wurde gefunden, dass die Grösse der Liposomen die Verweildauer (d.h. die Halbwertszeit) des β-Lactamasehemmers im Plasma beeinflusst. Zur Erzielung einer langen Verweildauer sollte der Durchmesser der Liposomen kleiner als etwa 250 nm sein. Erfindungsgemäss sind Liposomen mit einem Durchmesser von etwa 100 - 200 nm, insbesondere etwa 150 nm, bevorzugt.

Die erfindungsgemässen Liposomenlösungen können parenteral, z.B. intravenös, intramuskulär oder subcutan unter Berücksichtigung der für die darin enthaltenen Wirkstoffe bekannten Anwendungsrichtlinien verabreicht werden. Sie können Hilfsstoffe enthalten, die in pharmazeutischen Liposomenlösungen für die parenterale Verabreichung üblicherweise eingesetzt werden, insbesondere Stabilisatoren, wie Zucker, z.B. Sucrose oder Trehalose sowie Mittel zur Einstellung des pH und des osmotischen Drucks. Weiterhin können die erfindungsgemässen Liposomen-lösungen β-Lactamantibiotika, insbesondere Penicilline oder Cephalosporine, z.B. Piperacillin oder Apalcillin; oder wie z.B. Ceftriaxon, Ceftazid oder Cefoperazon; oder ein Carbapenem, wie Imipenem oder Meropenem; oder ein Monobactam, wie Aztreonam enthalten. Sie können ferner in lyophilisierter Form vorliegen.

Die erfindungsgemässen Liposomenlösungen können in an sich bekannter Weise hergestellt werden, beispielsweise dadurch, dass man aus den Liposomenbildnern mittels geeigneter Lösungsmittel, z.B. Chloroform/Methanol, einen lösungsmittelfreien Film hergestellt, diesen mit einer wässrigen Lösung des β-Lactamasehemmers hydratisiert und die so erhaltenen multilamellaren Vesikel durch Extrudieren in Liposomen der gewünschten Dimension überführt.

Die nachstehenden Beispiele erläutert die Erfindung weiter.

### Beispiel 1

50 mg eines Gemisches von Eilecithin (Lipoid E PC, Lipoid KG, Ludwigshafen, Deutschalnd), Phosphatidylglycerin (Lipoid PG, 16:0/16:0, Lipoid KG, Ludwigshafen, Deutschland) und Cholesterin im molaren Verhältnis von 10:1:5 wurden in einem Rundkolben in Chloroform/Methanol (1:1 Vol.-Teile) gelöst. Das Lösungsmittel wurde im rotierenden Kolben bei 40°C unter vermindertem Druck (zuletzt bei 0.01 mbar) entfernt. Der zurückbleibende Film wurde mit 1 ml einer 10%igen wässrigen Lösung von Tazobactam-Na versetzt und durch Behandlung in einem Vortex-Mischer hydratisiert. Die so erhaltenen multilamellaren, Tazobactam enthaltenden Vesikel wurden unter Stickstoffdruck (6-8 bar) dreimal durch ein 0.2 µm und ein- oder zweimal durch ein 0.1 µm - Polycarbonatfilter (Nucleopore, Pleasanton, CA., USA) gepresst. Die so erhaltenen Liposomen hatten einen mittleren Durchmesser von 150 nm (gemessen durch quasielastische Lichtstreuung mit einem Nano-Sizer PSM 78, Coulter Electronics, Krefeld, Deutschland). Analog können durch Wahl eines Filters grösserer (z.B. 0.4 µm) oder kleiner (z.B. 0.05 µm) Porenweite Liposomen mit grösserem oder kleinerem Durchmesser hergestellt werden.

### Beispiel 2

Männlichen Albinoratten wurden durch einen Jugularvenenkatheter Tazobactam-Liposomen (1 ml/kg, 5.8-10.4 mg Tazobactam/kg) verschiedener Grösse injiziert. Nach Spülen des Katheters mit physiologische Kochsalzlösung wurden zu verschiedenen Zeitpunkten durch den Katheter Plasmaproben entnommen, worauf das entnommene Plasma durch Kochsalzlösung ersetzt wurde. In den Proben wurde die Tazobactam-Konzentration mittels HPLC bestimmt. Die Resultate sind nachstehend wiedergegeben:

| Formulierung | Liposomen-Durchmesser [nm] | t 1/2 [h] | AUC [mg.h/ml] |
|---|---|---|---|
| Liposomen | 146 | 5.0±1.9 | 456.3±250.1 |
| Liposomen | 236 | 1.4±0.3 | 135.1± 62.5 |
| Wässrige Lösung | | 0.14 | 5.27 |

Die obigen Resultate zeigen, dass durch die Verabreichung in Form einer Liposomenlösung die Verweildauer des Wirkstoffs im Plasma verlängert war und dass dieser Effekt bei den Lösungen mit kleineren Liposomen stärker war als bei den grösseren Liposomen.

## Patentansprüche

1. Wässrige Liposomenlösung, enthaltend einen β-Lactamasehemmer und, gewünschtenfalls, einen Stabilisator.

2. Liposomenlösung gemäss Anspruch 1, worin der β-Lactamasehemmer Tazobactam, Sulbactam oder Clavulansäure, oder ein in der europäischen Patentpublikation A-0 508 234 beschriebener β-Lactamasehemmer; oder eine Verbindung der allgemeiner Formel I, 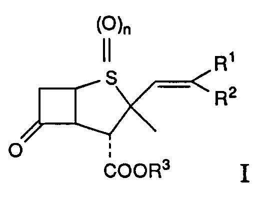 worin eines von R¹ und R² -COR⁴, -CN, -CH₂OR⁵ oder -SO₂R⁶ und das andere H, -COR⁴, -CN, -CH₂OR⁵ oder -SO₂R⁶ oder niederes Alkyl;
R³ = H, niederes Alkyl, Aryl-alkyl, Allyl oder einen in vivo abspaltbaren Rest;
R⁴ = H, niederes Alkyl, niederes Alkoxy, Benzyloxy, Amino oder niederes Alkylamino;
R⁵ = H oder -CONH₂;
R⁶ = niederes Alkyl und
n = 0, 1 oder 2 bedeuten
oder ein pharmazeutisch verträglicher Salz davon ist.

3. Liposomenlösung gemäss Anspruch 1 oder 2, worin der Stabilisator ein Zucker, wie Sucrose oder Trehalose ist.

4. Liposomenlösung gemäss den Ansprüchen 1-3, worin die Liposomen aus Phosphatidylcholin und Phosphatidylglycerin und, gegebenenfalls, Cholesterin bestehen.

5. Liposomenlösung gemäss Anspruch 4, worin das Molverhältnis von Phosphatidylcholin, Phosphatidylglycerin und Cholesterin 10:0-1:0-5 ist.

6. Liposomenlösung gemäss den Ansprüchen 1-5, worin die Liposomen im Mittel einen Durchmesser von 50-250 nm aufweisen.

7. Liposomenlösung gemäss Anspruch 6, worin die Liposomen im Mittel einen Durchmesser von etwa 150 nm aufweisen.

8. Liposomenlösung gemäss den Ansprüchen 1-7, worin zusätzlich ein β-Lactamantibiotikum anwesend ist.

9. Lyophilisiertes Liposomenpräparat gemäss der Ansprüchen 1-8.

10. Liposomenlösung gemäss den Ansprüchen 1-9, worin das β-Lactamantibiotikum ein Penicillin, Cephalosporin, Carbapenem oder Monobactam ist.
